# EUROPÄISCHE PATENTSCHRIFT

(11) **EP 0 413 092 B1**
(45) Veröffentlichungstag und Bekanntmachung des Hinweises auf die Patenterteilung: **22.06.1994**
(21) Anmeldenummer: 90109312.0
(22) Anmeldetag: 17.05.1990
(51) Int. Cl.: A61B 17/60

(54) **Osteosynthesehilfsmittel**
Auxiliary osteosynthesis system
Système auxiliaire d'ostéosynthèse

(30) Priorität: 16.08.1989 DE 3926893
(43) Veröffentlichungstag der Anmeldung: 20.02.1991
(73) Patentinhaber: Pennig, Dietmar, Dr. med., 50935 Köln (DE)
(72) Erfinder: Pennig, Dietmar, Dr. med., 50935 Köln (DE)
(74) Vertreter: Habbel, Hans-Georg, Dipl.-Ing.

(56) Entgegenhaltungen:
- DE-A- 3 701 533
- US-A- 4 336 796
- US-A- 4 407 277

## Beschreibung

Die Erfindung bezieht sich auf ein Osteosynthesehilfsmittel zur Fixation des Fußgelenkes in Verbindung mit einem äußeren Fixateur.

Äußere Fixateure, insbesondere metallische Hilfsmittel, dienen der Fixation einer Fraktur. Hierdurch soll die Festlegung der Frakturenden in der genau gewünschten und erforderlichen Stellung erfolgen und über lange Zeit diese Stellung aufrechterhalten werden. Solche heute üblichen, unilateralen Fixateure sind beispielsweise Gegenstand der US-PS 43 12 336.

Bei der Fixation der Unterschenkelknochen treten aber dadurch Probleme auf, daß der Fuß bei Flachlagerung des Patienten in eine Spitzfußstellung fällt, was es erforderlich macht, daß dieser Fuß ständig beübt werden muß. Ein Festlegen des Fußes in der gewünschten Normalstellung durch einen starren Verband ist nicht möglich, da hierdurch die Durchblutung gehindert wird und häufig auch Verletzungen im Bereich des Fußes vorliegen, die den Verband ausschließen. Liegen zudem Gehirnbeeinträchtigungen vor, darf ebenfalls ein Beüben des Fußes nicht stattfinden.

Aus der Literaturstelle "Manual der Osteosynthese", New York 1977, ist eine Dreieckverstrebung, d. h. eine sogenannte Rahmenmontage als Fixateur bekanntgeworden, die eine Platte zur Stützung des Fußes in Rechtwinkelstellung trägt. Eine Höhen-, Seiten- und Winkeleinstellung der Platte ist nicht vorgesehen und auch nicht möglich.

Der Erfindung liegt daher die Aufgabe zugrunde, ein zur Fixation des Fußgelenkes dienendes Osteosynthesehilfsmittel zu schaffen, das in Verbindung mit einem äußeren, unilateralen Fixateur die Möglichkeit gibt, den Fuß in einer beliebig wählbaren Stellung zu fixieren.

Diese der Erfindung zugrundeliegende Aufgabe wird durch die Lehre des Hauptanspruches gelöst.

Mit anderen Worten ausgedrückt wird ein Osteosynthesehilfsmittel vorgeschlagen, das an das üblicherweise untere Ende eines heute handelsüblichen, unilateralen Fixateurs, beispielsweise wie er in der US-PS 43 12 336 beschrieben ist, anschließt, wobei der Fixateur in beliebiger Lage angebracht sein kann, dann über eine in ihrer Länge einstellbare Längshaltestange zu einer Fußstützplatte führt, wobei die Fußstützplatte über eine Querhaltestange an der Längshaltestange anschließt. Dadurch, daß die Fußstützplatte über ein Kugelgelenk an der Querhaltestange anschließt, ist die Fußstützplatte um ihre Querachse neigbar und um ihre Längsachse einstellbar und kann damit der Ausrichtung des Fixateurs angepaßt werden und gleichzeitig auf die gewünschte beliebige Fußstellung des Patienten genau eingestellt werden.

In gewissen Einsatzfällen kommt es vor, daß die untere Klemmbacke des Fixateurs am Unterschenkel auch quer ausgerichtet angebracht werden muß. Um in einem solchen Fall auch das erfindungsgemäße Osteosynthesehilfsmittel anschließen zu können, wird ein 90° - Winkelstück vorgeschlagen, das zwischen das obere Ende der Längshaltestange und die Anschlußklammer eingeschaltet werden kann, so daß nunmehr auch die Anschlußklammer wiederum an den quergerichteten Fixateur angepaßt ist.

Um die Längshaltestange jeder beliebigen Länge anpassen zu können und damit jeder beliebigen Stellung des Fixateurs am Bein des Patienten, kann die Längshal testange aus mehreren Einzelteilen aufgebaut sein, die miteinander verschraubbar sind, so daß dadurch die Längenregulierung möglich ist.

Die erfindungsgemäß vorgesehenen Kugelgelenke können über einfache Knebelverschlüsse verstarrt werden, aber hier sind auch andere Verschlußmittel möglich, ohne daß hierauf die Erfindung in irgendeiner Weise beschränkt ist, aber es muß dafür Sorge getragen werden, daß nach Anschluß des erfindungsgemäßen Osteosynthesehilfsmittel s die einmal gewählte Stellung der einzelnen Teile verstarrt werden kann.

Ausführungsbeispiele der Erfindung werden nachfolgend anhand der Zeichnung erläutert. Die Zeichnung zeigt dabei in
- Fig. 1: schaubildlich ein Osteosynthesehilfsmittel, in
- Fig. 2: schematisch den Aufbau des Osteosynthesehilfsmittels gemäß der Erfindung und in
- Fig. 3: eine durch ein Winkelstück ergänzte Ausführungsform.

In der Zeichnung ist ein Endteil eines Fixateurs 1 dargestellt, von dem die Knochenschrauben oder Nägel 14 erkennbar sind. An dieses Endteil eines Fixateurs 1 schließt eine Anschlußklammer 2 an, die aus zwei Halbschalen 15 und 16 bestehen kann, wobei diese beiden Halbschalen 15, 16 über eine Schraubverbindung 17 fest miteinander verbindbar sind.

Die Anschlußklammer 2 schließt an das eine Ende einer Längshaltestange 3 an. Diese Längshaltestange 3 besteht bei dem dargestellten Ausführungsbeispiel aus drei Teilen 10, 11 und 12, wobei die Teile 12 und 11 miteinander verschraubbar sind. Wird das Teil 11 nicht eingesetzt, ist erkennbar, daß das Teil 10 unmittelbar an das Teil 12 anschließen kann. Außerdem wird die Längenverstellbarkeit der Längshaltestange 3 dadurch ermöglicht, daß das Teil 10 an seinem Ende 18 muffenartig ausgebildet ist und hier mit einem Klemmverschluß 19 ausgerüstet ist.

Das andere Ende der Längshaltestange 3 trägt eine Querhaltestange 4, die ebenfalls längenverstellbar ausgebildet ist, dadurch, daß diese Querhaltestange 4 aus zwei Stangenteilen 20 und 21 besteht, wobei das Stangenteil 20 ähnlich wie das Stangenteil 10 eine Muffe aufweist, die über Klemmittel an dem Teil 21 festgelegt werden kann.

Die Querhaltestange 4 trägt eine Fußstützplatte 5, die eine Polsterung 22 besitzt, wobei diese Polsterung 22 zur Fußsohle des Patienten hin gerichtet ist. Die Querhaltestange 4 schließt an die Fußstützplatte 5 über ein Kugelgelenk 8 an, das über den Knebelverschluß 23 verriegelbar ist. Über dieses Kugelgelenk 8 kann die Fußstützplatte 5 um mehrere Achsen gedreht werden, so daß sie der Stellung und Neigung des Fußes des Patienten anpaßbar ist.

Die Querhaltestange 4 schließt aber auch über ein Kugelgelenk 7 an die Längshaltestange 3 an und die Längshaltestange 3 schließt wiederum über ein Kugelgelenk 6 an die Anschlußklammer 2 an. Alle diese Kugelgelenke 6, 7, 8 sind verstarrbar.

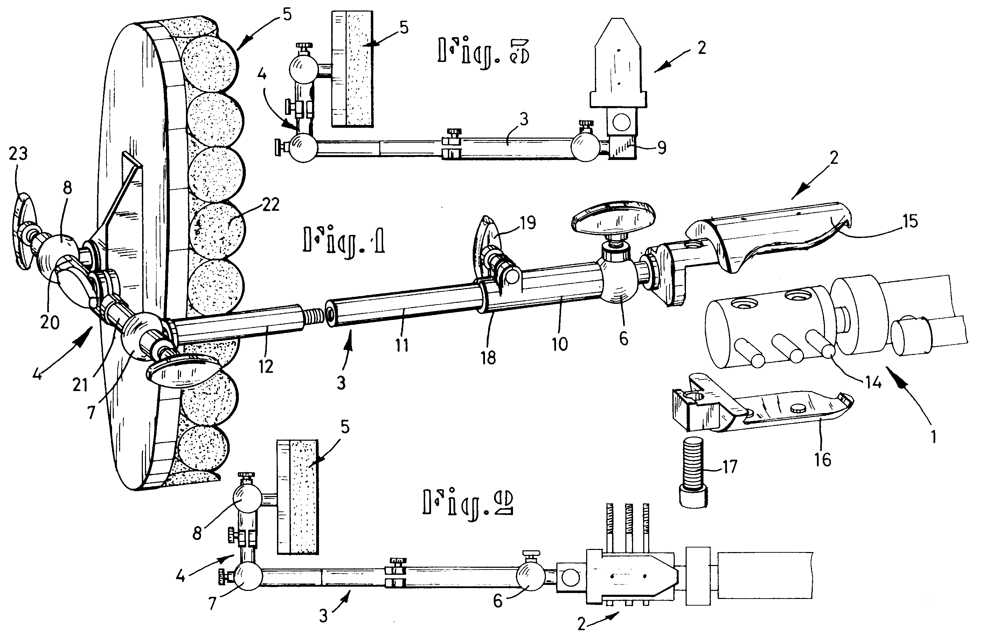

Dieser vorbeschriebene Aufbau geht schematisch deutlicher aus Fig. 2 hervor.

In Fig. 3 ist dargestellt, daß es möglich ist, zwischen das der Anschlußklammer 2 zugewandte Ende der Haltestange 3 ein "90° - Winkelstück" 9 zwischenzuschließen, so daß es möglich ist, trotz einer parallelen Ausrichtung der Längshaltestange 3, beispielsweise zum Schienbein des Patienten, die Längshaltestange 3 an einen Fixateur 1 anzuschließen, der quer zur Längsachse der Unterschenkelknochen angeordnet ist.

Es sei darauf hingewiesen, daß die Ausbildung der Anschlußklammer 2 eine speziale Anpassung an den jeweils eingesetzten Fixateur notwendig macht und daß zur Verdeutlichung der Erfindung in der vorliegenden Beschreibung und Zeichnung eine Anschlußklammer 2 dargestellt ist, die in Verbindung mit einem aus der US-PS 43 12 336 bekannten Fixateur einsetzbar ist.

## Patentansprüche

1. Osteosynthesehilfsmittel zur Fixation des Fußgelenkes in Verbindung mit einem äußeren Fixateur, gekennzeichnet durch
a) eine Anschlußklammer (2) zur Festlegung des Hilfsmittels an einen Fixateur (1),
b) eine längenveränderliche Längshaltestange (3), die einenendes an der Anschlußklammer (2) anschließt und anderenendes mittelbar eine Fußstützplatte (5) trägt,
c) eine Querhaltestange (4), die einenendes die Fußstützplatte (5) trägt und anderenendes an der Längshaltestange (3) anschließt,
d) Kugelgelenke (6, 7, 8) als Anschlußstellen zwischen
1) der Längshaltestange (3) und der Anschlußklammer (2),
2) der Längshaltestange (3) und der Querhaltestange (4),
3) der Querhaltestange (4) und der Fußstützplatte (5),
wobei die Kugelgelenke (6, 7, 8) arretierbar sind.

2. Osteosynthesehilfsmittel nach Anspruch 1, dadurch gekennzeichnet, daß zwischen der Anschlußklammer (2) und der Längshaltestange (3) ein "90° - Winkelstück" (9) anbringbar ist.

3. Osteosynthesehilfsmittel nach Anspruch 1 oder 2, dadurch gekennzeichnet, daß die Querhaltestange (4) längenveränderlich ist.

4. Osteosynthesehilfsmittel nach einem oder mehreren der vorhergehenden Ansprüche, dadurch gekennzeichnet, daß die Längshaltestange (3) aus mehreren miteinander verbindbaren Teilen (10, 11, 12) besteht und stufenlos längenverstellbar ist.

5. Osteosynthesehilfsmittel nach einem oder mehreren der vorhergehenden Ansprüche, dadurch gekennzeichnet, daß die Fußstützplatte (5) gepolstert ist.

6. Osteosynthesehilfsmittel nach Anspruch 1, dadurch gekennzeichnet, daß die Anschlußklammer (2) aus zwei fest miteinander verbindbaren Halbschalen (15, 16) besteht.

## Claims

1. An auxiliary osteosynthesis agent for fixing the foot joint in conjunction with an external fixer, characterized by
a) a connector clamp (2) for fixing the auxiliary agent to a fixer (1),
b) a length-adjustable longitudinal stay bar (3), which at one end is connected to the connector clamp (2) and at the other end indirectly carries a foot support plate (5),
c) a transverse stay bar (4), which at one end carries the foot support plate (5) and at the other end is connected to the longitudinal stay bar (3),
d) ball-and-socket joints (6, 7, 8) as connection points between
1) the longitudinal stay bar (3) and the connector clamp (2),
2) the longitudinal stay bar (3) and the transverse stay bar (4),
3) the transverse stay bar (4) and the foot support plate (5),
the ball-and-socket joints (6, 7, 8) being arrestable.

2. An auxiliary osteosynthesis agent according to claim 1, characterized in that a "90° corner piece" (9) can be inserted between the connector clamp (2) and the longitudinal stay bar (3).

3. An auxiliary osteosynthesis agent according to claim 1 or claim 2, characterized in that the transverse stay bar (4) is of adjustable length.

4. An auxiliary osteosynthesis agent according to any one of the preceding claims, characterized in that the longitudinal stay bar (3) consists of a plurality of interconnectable parts (10, 11, 12) and is capable of continuous length adjustment.

5. An auxiliary osteosynthesis agent according to any one of the preceding claims, characterized in that the foot support plate (5) is padded.

6. An auxiliary osteosynthesis agent according to claim 1, characterized in that the connector clamp (2) consists of two half-shells (15, 16) firmly connectable together.

## Revendications

1. Auxiliaire d'ostéosynthèse pour l'immobilisation du cou-de-pied, en liaison avec un élément d'immobilisation extérieur, caractérisé par :
a) un manchon de raccordement (2) pour la fixation de l'auxiliaire sur un élément d'immobilisation (1),
b) une tige longitudinale de maintien (3) de longueur variable, qui se raccorde par une extrémité au manchon de raccordement (2) et qui porte indirectement, à son autre extrémité, une plaque support de pied (5),
c) une tige transversale de maintien (4) qui porte, à une extrémité, la plaque support de pied (5) et qui se raccorde, par son autre extrémité, à la tige longitudinale de maintien (3),
d) des articulations sphériques (6, 7, 8) en tant que points de raccordement entre
1) la tige longitudinale de maintien (3) et le manchon de raccordement (2),
2) la tige longitudinale de maintien (3) et la tige transversale de maintien (4),
3) la tige transversale de maintien (4) et la plaque support de pied (5),
les articulations sphériques (6, 7, 8) pouvant être bloquées en position.

2. Auxiliaire d'ostéosynthèse selon la revendication 1, caractérisé en ce qu'une "pièce coudée à 90°" (9) peut être montée entre le manchon de raccordement (2) et la tige longitudinale de maintien (3).

3. Auxiliaire d'ostéosynthèse selon la revendication 1 ou 2, caractérisé en ce que la tige transversale de maintien (4) est réglable en longueur.

4. Auxiliaire d'ostéosynthèse selon l'une ou plusieurs des revendications précédentes, caractérisé en ce que la tige longitudinale de maintien (3) consiste en plusieurs parties (10, 11, 12) propres à être fixées les unes aux autres et en ce qu'elle est à réglage continu en longueur.

5. Auxiliaire d'ostéosynthèse selon l'une ou plusieurs des revendications précédentes, caractérisé en ce que la plaque support de pied (5) est rembourrée.

6. Auxiliaire d'ostéosynthèse selon la revendication 1, caractérisé en ce que le manchon de raccordement (2) consiste en deux demi-coquilles (15, 16) propres à être fermement fixées l'une à l'autre.
